# EUROPEAN PATENT APPLICATION

(11) **EP 2 286 726 A2**
(43) Date of publication of application: **23.02.2011**
(21) Application number: 09742851.0
(22) Date of filing: 07.05.2009
(51) Int. Cl.: A61B 6/00

(54) **HIGH-VOLTAGE POWER SUPPLY APPARATUS AND METHOD FOR USE WITH DIGITAL FLAT PANEL X-RAY DETECTORS**

(30) Priority: 09.05.2008 KR 20080043602
(71) Applicant: Drtech Corp., Seongnam-si Gyeonggi-do 463-954 (KR)
(72) Inventor: LEE, Sang-il, Gunpo-si Gyeonggi-do 435-050 (KR); MOON, Beom-jin, Suwon-si Gyeonggi-do 443-737 (KR); YOON, Jung-kee, Anyang-si Gyeonggi-do 431-070 (KR)
(74) Representative: Herzog, Markus
(86) International application number: PCT/KR2009/002407
(87) International publication number: WO 2009/136745

(57) **Abstract**

Provided are a high-voltage power supply apparatus and method for supplying high-voltage power to a digital flat panel X-ray detector to accelerate the separation of charges ionized upon the irradiation of the digital flat panel X-ray detector with X-rays. The high-voltage power supply apparatus includes a power source outputting high-voltage power; a path determiner providing a supply path for supplying the high-voltage power to the digital flat panel X-ray detector and a short-circuit path for short-circuiting the high-voltage power; and a path controller determining the supply path and the short-circuit path. Therefore, it is possible to prevent an overshoot of the waveform of the high-voltage power. In addition, it is possible to improve the uniformity of a radiation image and reduce the time taken to obtain a radiation image by quickly discharging charges when the high-voltage power is short-circuited.

## Description

### Technical Field

The present invention relates to a digital flat panel X-ray detector, and more particularly, to a high-voltage power supply apparatus and method for supplying high-voltage power to a digital flat panel X-ray detector to accelerate the separation and movement of charges ionized upon the irradiation of the digital flat panel X-ray detector with X-rays.

### Background Art

X-ray imaging systems for medical applications obtain a radiation image by taking an image of a subject using a film and developing the film. Digital flat panel X-ray detectors capable of obtaining digital images using thin-film transistors (TFTs) have recently been developed.

A typical digital flat panel X-ray detector includes a 2-dimensional array of photosensitive pixels that are sensitive to X-rays and thus generate charges upon exposure to X-rays. The typical digital flat panel X-ray detector may also include a charge accumulator accumulating the charges therein and TFTs analyzing the charges. More specifically, when the digital flat panel X-ray detector is irradiated with X-rays, amorphous silicon in the digital flat panel X-ray detector may be ionized, and positive and negative charges generated upon the ionization of the amorphous silicon may move toward a charge collection electrode and a top electrode, respectively, in the digital flat panel X-ray detector. Then, the TFTs may read the charges collected and accumulated in the charge collection electrode.

In order to improve the moving speed of the positive and negative charges, a high voltage may be applied to the digital flat panel X-ray detector. However, typical high-voltage power supply apparatuses simply supply high-voltage power to a digital flat panel X-ray detector or short-circuit the high-voltage power without a requirement of additional control equipment, thus causing an overshoot of the waveform of the high-voltage power. In addition, since it generally takes time for the high-voltage power to decrease below a predefined level, typical high-voltage power supply apparatuses are generally prone to delays in reading charges.

### Technical Problem

The following description relates to a high-voltage power supply apparatus and method for supplying high-voltage power to a digital flat panel X-ray detector for accelerating the separation and movement of charges ionized upon the irradiation of the digital flat panel X-ray detector with X-rays, the high-voltage supply apparatus and method being capable of effectively controlling the waveform of the high-voltage power by providing two separate paths as a supply path for supplying the high-voltage power and a short-circuit path for short-circuiting the high-voltage power.

The following description also relates to a high-voltage power supply apparatus and method for use with a digital flat panel X-ray detector, which can reduce the time taken to obtain a radiation image by quickly connecting an end of a short-circuit path for short-circuiting high-voltage power to a ground source when the high-voltage power is short-circuited, and thus quickly discharging charges remained in the digital flat panel X-ray detector.

### Technical Solution

The present invention provides a high-voltage power supply apparatus for supplying high-voltage power to a digital flat panel X-ray detector to accelerate the separation and movement of charges ionized upon the irradiation of the digital flat panel X-ray detector with X-rays, the high-voltage power supply apparatus including a power source outputting high-voltage power; a path determiner providing a supply path for supplying the high-voltage power to the digital flat panel X-ray detector and a short-circuit path for short-circuiting the high-voltage power; and a path controller determining the supply path and the short-circuit path.

The path determiner may provide two separate paths as the supply path and the short-circuit path and the supply path, and the short-circuit path may be connected in parallel to the power source.

An end of the short-circuit path may be connected to a ground source, and charges remained in the digital flat panel X-ray detector may be discharged via the short-circuit path when the high-voltage power is short-circuited.

The path determiner may include a first path including a resistor, the first path being used as the supply path; and a second path including a switch, the second path being used as the short-circuit path.

The present invention provides a high-voltage power supply method for supplying high-voltage power to a digital flat panel X-ray detector to accelerate the separation and movement of charges ionized upon the irradiation of the digital flat panel X-ray detector with X-rays, the high-voltage power supply method including determining a supply path for supplying high-voltage power to the digital flat panel X-ray detector and a short-circuit path for short-circuiting the high-voltage power; and supplying the high-voltage power to the X-ray detector using the supply path and the short-circuit path.

Additional features of the invention will be set forth in the description which follows, and in part will be apparent from the description, or may be learned by practice of the invention.

### Advantageous Effects

According to the present invention, it is possible to prevent an overshoot of the waveform of high-voltage power by controlling the rising time of the high-voltage power when supplying the high-voltage power, and controlling the falling time of the high-voltage power when short-circuiting the high-voltage power. In addition, it is possible to improve the uniformity of a radiation image and reduce the time taken to obtain a radiation image by quickly discharging charges when the high-voltage power is short-circuited.

### Description of Drawings

The accompanying drawings, which are included to provide a further understanding of the invention and are incorporated in and constitute a part of this specification, illustrate embodiments of the invention, and together with the description serve to explain the principles of the invention.
FIG. 1 is a diagram showing an exemplary high-voltage power supply apparatus;
FIG. 2 is a detailed diagram showing another exemplary high-voltage power supply apparatus;
FIG. 3 is a diagram showing an example of the timings of a switch operation, a high-voltage supply operation and a data read operation performed by the high-voltage power supply apparatus shown in FIG. 2;
FIG. 4 is a diagram showing another example of the timings of the switch operation, the high-voltage supply operation and the data read operation performed by the high-voltage power supply apparatus shown in FIG. 2;
FIG. 5 is a diagram showing another exemplary high-voltage power supply apparatus;
FIG. 6 is a diagram showing an example of the timings of a switch operation, a high-voltage supply operation and a data read operation performed by the high-voltage power supply apparatus shown in FIG. 5; and
FIG. 7 is a flowchart of an exemplary high-voltage power supply method.

### Best Mode

The present invention discloses a high-voltage power supply apparatus for supplying high-voltage power to a digital flat panel X-ray detector to accelerate the separation and movement of charges ionized upon the irradiation of the digital flat panel X-ray detector with X-rays, the high-voltage power supply apparatus including a power source outputting high-voltage power; a path determiner providing a supply path for supplying the high-voltage power to the digital flat panel X-ray detector and a short-circuit path for short-circuiting the high-voltage power; and a path controller determining the supply path and the short-circuit path.

It is to be understood that both the foregoing general description and the following detailed description are exemplary and explanatory and are intended to provide further explanation of the invention as claimed.

### Mode for Invention

The invention is described more fully hereinafter with reference to the accompanying drawings, in which exemplary embodiments of the invention are shown. This invention may, however, be embodied in many different forms and should not be construed as limited to the embodiments set forth herein. Rather, these embodiments are provided so that this disclosure is thorough, and will fully convey the scope of the invention to those skilled in the art. In the drawings, the size and relative sizes of layers and regions may be exaggerated for clarity. Like reference numerals in the drawings denote like elements.

It will be understood that when an element or layer is referred to as being "on" or "connected to" another element or layer, it can be directly on or directly connected to the other element or layer, or intervening elements or layers may be present. In contrast, when an element is referred to as being "directly on" or "directly connected to" another element or layer, there are no intervening elements or layers present.

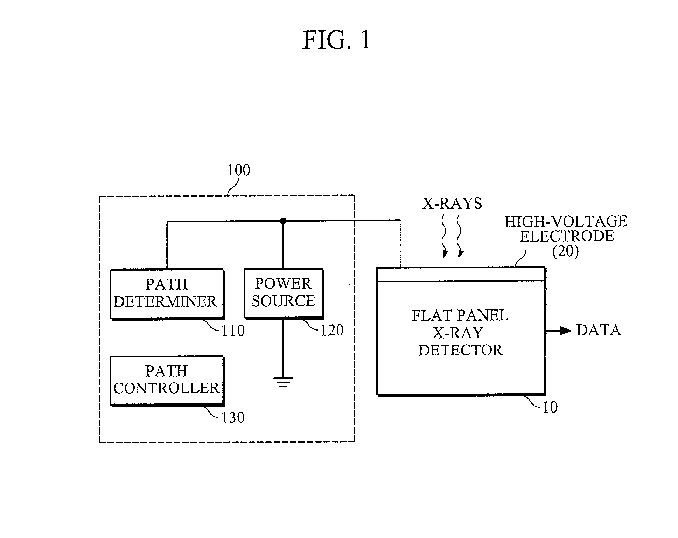

FIG. 1 is a diagram showing an exemplary high-voltage power supply apparatus 100. Referring to FIG. 1, the high-voltage power supply apparatus 100 may include a path determiner 110, a power source 120, and a path controller 130. When a flat panel X-ray detector 10 is irradiated with X-rays, amorphous silicon in the flat panel X-ray detector 10 may be ionized, and thus, positive and negative charges generated in the flat panel X-ray detector 10 may be separated from each other. Then, the negative charges move toward a top electrode, and the positive charges may move toward a charge collection electrode. In this case, in order to accelerate the moving speed of the positive and negative charges, the high-voltage power supply apparatus 100 may supply high-voltage power to the flat panel X-ray detector 10 via a high-voltage electrode 20.

The power source 120 may output high-voltage power. The path determiner 110 may form a supply path for supplying the high-voltage power output by the power source 120 to the flat panel X-ray detector 10, and a short-circuit path for short-circuiting the high-voltage power output by the power source 120. That is, the high-voltage power output by the power source 120 may not only be supplied to the flat panel X-ray detector 10 but also flow into the path determiner 110. Thus, the rising time or falling time of the high-voltage power supplied to the flat panel X-ray detector 10 may vary according to whether the supply path or the short-circuit path is formed by the path determiner 110.

The supply path and the short-circuit path may be separate from each other, and may be connected in parallel to the power source 120, as shown in FIG. 1. Since an end of the short-circuit path is connected to a ground source, it is possible to quickly discharge charges, if any, remained in the flat panel X-ray detector 10 when the high-voltage power output by the power source 120 is short-circuited.

The path controller 130 may determine whether to provide the supply path or the short-circuit path. That is, the path controller 130 may control the supply of high-voltage power and the short-circuiting of high-voltage power to be performed following different paths. The path controller 130 may control the supply path and the short-circuit path to be selectively provided according to the characteristic values of elements (such as switching devices and resistors) that form the supply path or the short-circuit path. Various embodiments of the path determiner 110 and a switching timing will hereinafter be described in detail.

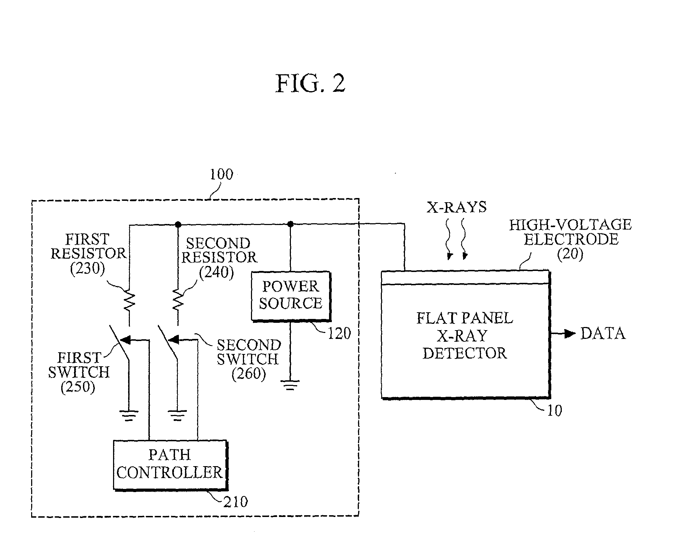

FIG. 2 is a detailed diagram showing another high-voltage power supply apparatus 100. Referring to FIG. 2, a path determiner 110 may include a first path formed by a first resistor 230 and a first switch 250 and a second path formed by a second resistor 240 and a second switch 260. A path controller 210 may control the turning on or off of the first and second switches 250 and 260 and may thus control the first and second paths.

The first path may be used as a path for supplying high-voltage power output by a power source 220 to a flat panel X-ray detector 10. That is, when the high-voltage power is supplied to the flat panel X-ray detector 10, the first switch 250 may be turned on, and thus, the high-voltage power may flow through the first resistor 230 and the first switch 250. When the high-voltage power reaches a predefined value, the first switch 250 may be turned off, and may then remain turned off for a predefined amount of time.

The second path may be used as a path for discharging the voltage remained in the flat panel X-ray detector 10 after the short-circuiting of the high-voltage power. That is, when the supply of high-voltage power by the power source 220 is cut off, the second switch 260 may be turned on, and thus, the voltage remained in the flat panel X-ray detector 10 may be discharged via the second resistor 240 and the second switch 260. Thereafter, when the high-voltage power output by the power source 220 is stabilized, data may be read from the flat panel X-ray detector 10.

The first switch 250 and the second resistor 240 may be optional. That is, the first path may be formed solely by the first resistor 2302, and the second path may be formed solely by the second switch 260. The resistances of the first and second resistors 230 and 240 may vary. The rising and falling times of the high-voltage power output by the power source 220 may be determined by the resistances of the first and second resistors 230 and 240.

The first and second switches 250 and 260 may be relays, solid state relays (SSRs), field effect transistors (FETs), or insulated gate bipolar transistors (IGBTs).

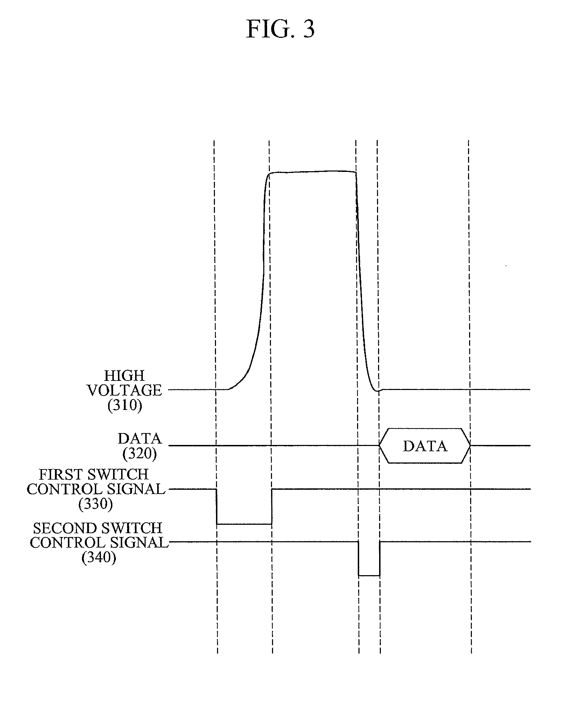

FIG. 3 is a diagram showing an example of the timings of a switch operation, a high-voltage supply operation and a data read operation performed by the high-voltage power supply apparatus 100 shown in FIG. 2. Referring to FIGS. 2 and 3, the rising and falling times of a high-voltage signal 310 may be determined by a first switch control signal 330, which is active low, and a second switch control 240, which is active low. Data 320 may be read after the falling time of the high-voltage signal.

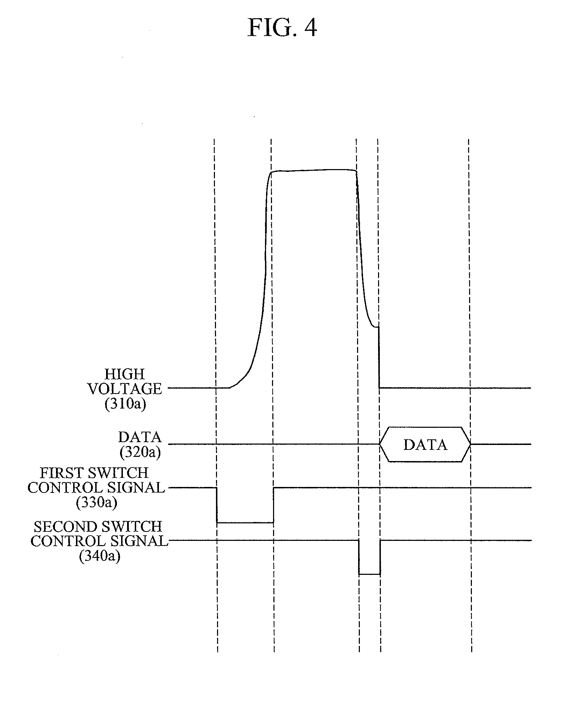

FIG. 4 is a diagram showing another example of the timings of the switch operation, the high-voltage supply operation and the data read operation performed by the high-voltage power supply apparatus 100 shown in FIG. 2. Referring to FIG. 4, the time when a second switch control signal 340a is turned on or off may vary in response to a variation in the falling time of a high-voltage signal 310a. The time taken to read data 320a may decrease according to the time when the second switch control signal 340a is turned on or off.

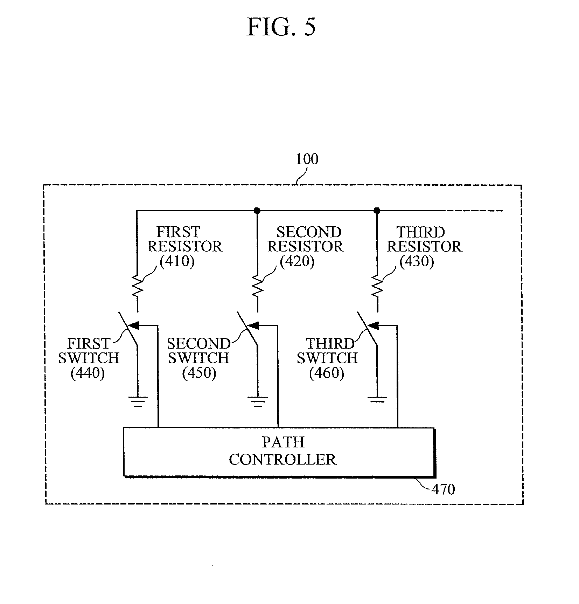

FIG. 5 is a diagram showing another exemplary high-voltage power supply apparatus 100. Referring to FIG. 5, a supply path for supplying high-voltage power and a short-circuit path for short-circuiting high-voltage power may be established in various manners. For example, three paths may be provided in the high-voltage power supply apparatus 100: a first path formed by a first resistor 410 and a first switch 440, a second path formed by a second resistor 420 and a second switch 450, and a third path formed by a third resistor 430 and a third switch 460. A path controller 470 may control the turning on or off of the first, second and third switches 440, 450 and 460 and may thus determine which of the first, second and third paths is to be used as the supply path or the short-circuit path.

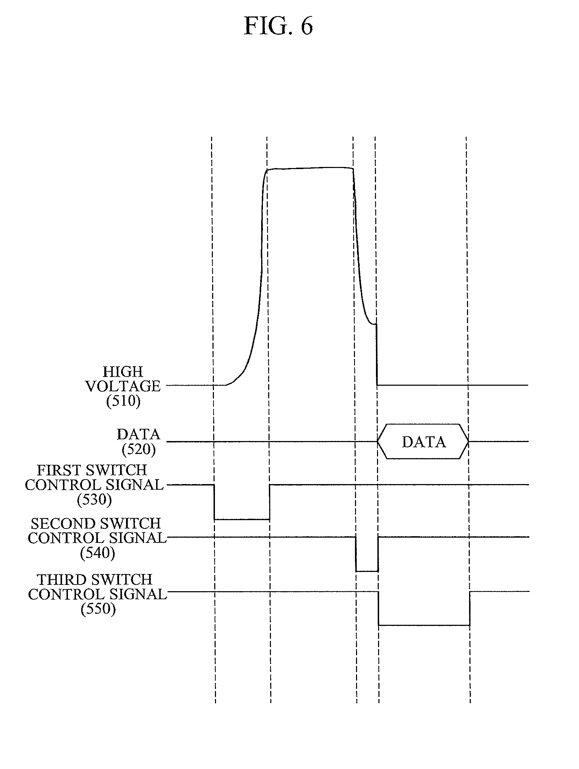

FIG. 6 is a diagram showing an example of the timings of a switch operation, a high-voltage supply operation and a data read operation performed by the high-voltage supply apparatus 100 shown in FIG. 5. Referring to FIG. 6, the level of a high-voltage signal 510 may gradually increase to a predefined level while a first switch control signal 530 is turned on. The level of the high-voltage signal 510 may decrease while a second switch control signal 540 is turned on. Data 520 may be read while a third switch control signal 550 is turned on.

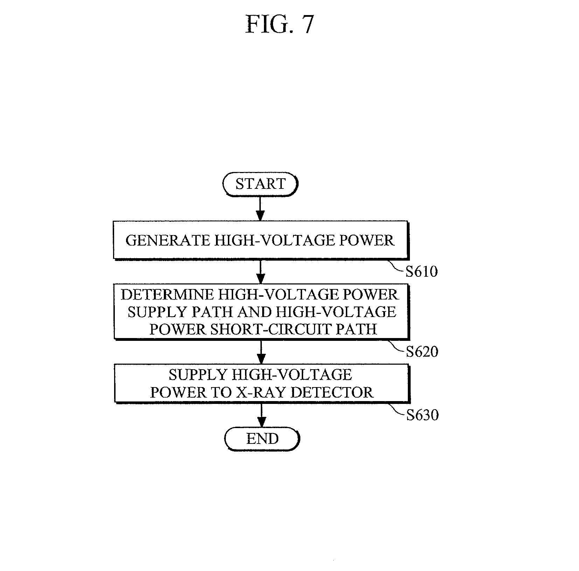

FIG. 7 is a flowchart of an exemplary high-voltage power supply method. Referring to FIG. 7, high-voltage power may be generated (S610). Thereafter, a supply path for supplying the high-voltage power and a short-circuit path for short-circuiting the high-voltage power may be determined (S620). The supply path and the short-circuit path may be connected in parallel to the power source 120. An end of the short-circuit path may be connected to a ground source, and thus, discharges remained in the flat panel X-ray detector may thus be able to be effectively discharged when the high-voltage power is short-circuited.

Thereafter, the high-voltage power may be supplied to the flat panel X-ray detector using the supply path and the short-circuit path (S630).

The present invention can be implemented as a computer program. Codes and code segments of the computer program may readily be inferred by programmers of ordinary skill in the art to which the present invention pertains. The computer program may be recorded on a computer readable medium and may be read and executed by a computer. Examples of the computer readable medium include, but are not limited to, a magnetic storage medium, an optical recording medium and a carrier wave medium.

It will be apparent to those skilled in the art that various modifications and variation can be made in the present invention without departing from the spirit or scope of the invention. Thus, it is intended that the present invention cover the modifications and variations of this invention provided they come within the scope of the appended claims and their equivalents.

### INDUSTRIAL APPLICABILITY

The present invention can be efficiently applied to a digital flat panel X-ray detector and can be used in the field of digital X-ray imaging without a requirement of films.

## Claims

1. A high-voltage power supply apparatus for supplying high-voltage power to a digital flat panel X-ray detector to accelerate the separation and movement of charges ionized upon the irradiation of the digital flat panel X-ray detector with X-rays, the high-voltage power supply apparatus comprising:
a power source outputting high-voltage power;
a path determiner providing a supply path for supplying the high-voltage power to the digital flat panel X-ray detector and a short-circuit path for short-circuiting the high-voltage power; and
a path controller determining the supply path and the short-circuit path.

2. The high-voltage power supply apparatus of claim 1, wherein the path determiner provides two separate paths as the supply path and the short-circuit path, and the supply path and the short-circuit path are connected in parallel to the power source.

3. The high-voltage power supply apparatus of claim 1 or 2, wherein an end of the short-circuit path is connected to a ground source, and charges remained in the digital flat panel X-ray detector are discharged via the short-circuit path when the high-voltage power is short-circuited.

4. The high-voltage power supply apparatus of claim 1, wherein the path determiner comprises:
a first path including a resistor, the first path being used as the supply path; and
a second path including a switch, the second path being used as the short-circuit path.

5. The high-voltage power supply apparatus of claim 4, wherein the first path further includes a switch and the second path further includes a resistor.

6. The high-voltage power supply apparatus of claim 4 or 5, wherein the path controller controls the switches of the first and second paths.

7. The high-voltage power supply apparatus of claim 4 or 5, wherein resistances of the resistors of the first and second paths are variable.

8. The high-voltage power supply apparatus of claim 4 or 5, wherein the path determiner further comprises a third path, the third path being used as the supply path or the short-circuit path.

9. The high-voltage power supply apparatus of claim 4 or 5, wherein the switches of the first and second paths include relays, solid state relays (SSRs), field effect transistors (FETs), or insulated gate bipolar transistors (IGBTs).

10. A high-voltage power supply method for supplying high-voltage power to a digital flat panel X-ray detector to accelerate the separation and movement of charges ionized upon the irradiation of the digital flat panel X-ray detector with X-rays, the high-voltage power supply method comprising:
determining a supply path for supplying high-voltage power to the digital flat panel X-ray detector and a short-circuit path for short-circuiting the high-voltage power; and
supplying the high-voltage power to the X-ray detector using the supply path and the short-circuit path.

11. The high-voltage power supply method of claim 10, wherein the determining of the supply path and the short-circuit path comprises providing two separate paths as the supply path and the short-circuit path, the supply path and the short-circuit path being connected in parallel to the power source.

12. The high-voltage power supply method of claim 10 or 11, wherein an end of the short-circuit path is connected to a ground source, and charges remained in the digital flat panel X-ray detector are discharged via the short-circuit path when the high-voltage power is short-circuited.
